# EUROPÄISCHE PATENTANMELDUNG

(11) **EP 4 461 291 A1**
(43) Veröffentlichungstag der Anmeldung: **13.11.2024**
(21) Anmeldenummer: 24154873.4
(22) Anmeldetag: 31.01.2024
(51) Int. Cl.: A61K 8/46, A61K 8/73, A61K 8/891, A61K 8/898, A61Q 5/02, A61Q 5/12

(54) **HAARPFLEGESHAMPOO ENTHALTEND EIN SILOXANPOLYMER MIT MINDESTENS DREI UNTERSCHIEDLICHEN FUNKTIONELLEN GRUPPEN**

(30) Priorität: 11.05.2023 DE 102023204380
(71) Anmelder: Henkel AG & Co. KGaA, 40589 Düsseldorf (DE)
(72) Erfinder: Scheele, Soeren, 25421 Pinneberg (DE); Westphal, Petra, 21629 Neu Wulmstorf (DE); Schroeder, Thomas, 22395 Hamburg (DE); Mette, Manuela, 23923 Kleinfeld (DE)

(57) **Zusammenfassung**

Die vorliegende Erfindung betrifft ein haarpflegendes kosmetisches Reinigungsmittel mit verbesserter Silikonabscheidung auf den Haarfasern, das neben mindestens einem anionischen Tensid und mindestens einem kationischen Polymer eine spezifische Mischung zweier unterschiedlicher Silikone enthält.

## Beschreibung

Die vorliegende Erfindung betrifft ein kosmetisches Reinigungsmittel, das neben mindestens einem anionischen Tensid und mindestens einem kationischen Polymer eine spezifische Silikonmischung enthält. Die Erfindung betrifft weiterhin die Verwendung des Reinigungsmittels zur Reinigung und Pflege von Haaren sowie ein Verfahren zur Reinigung und Pflege von Haaren unter Verwendung des Reinigungsmittels.

Kosmetische Reinigungsmittel enthalten üblicherweise Tenside bzw. Tensidgemische, um den Schmutz effektiv von der Anwendungsoberfläche zu entfernen. Tenside bzw. Tensidgemische gewährleisten die Emulgierung oder Solubilisierung von Verunreinigungen wie beispielsweise Fett, Talg, Partikeln, Stylingmittelrückständen, und entfernen diese durch nachfolgendes Spülen mit Wasser von den Haaren.

Während der Reinigung werden aus den Haarfasern und/oder der Kopfhaut jedoch auch Lipide und/oder Proteine entfernt, wodurch insbesondere bei häufiger Reinigung eine unerwünschte Schädigung der Haarstruktur und/oder ein Austrocknen der Kopfhaut auftreten kann. Die Folge davon sind beispielsweise ein für den Endverbraucher unangenehmes, spannendes (Kopf)hautgefühl sowie trockene, fliegende und/oder stumpfe Haare.

Zur Vermeidung des Auftretens der genannten Nachteile müssen geeignete Pflege- und Wirkstoffe den Haaren und/oder der Kopfhaut nach der Reinigung wieder zugeführt werden. Dafür werden beispielsweise Haarpflegemittel wie Haarkuren, Spülungen oder Haarpflegelotionen angewendet. Für den täglichen Gebrauch werden von den Verbrauchern aus Zeit- und Kostengründen oftmals Reinigungsmittel bevorzugt, welche die gleichzeitige Reinigung und Pflege von Haaren gewährleisten. Solche sogenannten 2in1- oder 3in1-Präparate enthalten neben Reinigungstensiden üblicherweise Fettstoffe, wie beispielsweise Silikone, sowie kationische Polymere, welche die Abscheidung von Fettstoffen auf den Haarfasern unterstützen.

In EP 811371A2 wird ein Haarpflegeshampoo offenbart, welches neben einer Waschbase ein Konditionierungssystem, umfassend ein kationisches Polymer, ein Aminosilikon sowie ein davon verschiedenes Silikon mit einer Viskosität < 100 Pas enthält.

Die durchschnittlich kurze Einwirkungszeit eines Shampoos auf den Haaren sowie die Tatsache, dass ein Großteil der in Shampoos enthaltenden Wirkstoffe beim Abspülvorgang von den Haaren wieder entfernt wird, führt zu einem nicht immer zufriedenstellenden oder ausreichenden Pflegeeffekt. Eine Erhöhung der Einsatzmengen (haar)konditionierender Inhaltsstoffe bietet keine akzeptable Lösung, denn aus wirtschaftlichen, nachhaltigen und leistungsbezogenen Gründen ist es nicht wünschenswert, eine noch größere Menge an wertvollen Rohmaterialien ohne nennenswerten Mehrwert ungenutzt abzuspülen. Darüber hinaus wären für eine ausreichende Stabilisierung höherer Mengen an (haar)konditionierenden Inhaltsstoffen auch höhere Mengen an Wirkstoffen zur Stabilisierung der Zusammensetzungen erforderlich, was wiederum dazu führen würde, dass wesentliche Mengen an Rohmaterialien ungenutzt abgespült werden müssten.

Ein nicht ausreichender Pflegeeffekt kann sich beispielsweise dadurch bemerkbar machen, dass die durch mechanische Behandlung der Haarfasern (wie Kämmen, Bürsten, Trockenrubbeln) erzeugte Haarreibung unerwünscht hoch ist, wodurch Haarschädigungen verursacht werden können. Generell gilt: je glatter die Haarfasern sind, desto geringer ist die Reibung bei mechanischer Behandlung.

Der vorliegenden Erfindung lag daher die Aufgabe zugrunde, verbesserte kosmetische Reinigungsmittel bereitzustellen, die eine gute Waschleistung sowie sehr gute pflegende Eigenschaften - insbesondere sehr gute Haarpflegeeigenschaften - vereinen. Insbesondere sollten die Reinigungsmittel die Abscheidung höherer Mengen an Pflegewirkstoffen auf der Haaroberfläche und/oder der Kopfhaut gewährleisten, ohne dass die Gesamtmenge an Pflegewirkstoffen in den Reinigungsmitteln signifikant gesteigert werden muss.

Behandelte Haarfasern sollten zudem gute Kämmbarkeitseigenschaften und mehr Glätte aufweisen. Des Weiteren sollen die Reinigungsmittel gute Schaumeigenschaften aufweisen und lagerstabil sein.

Überraschenderweise wurde gefunden, dass die Silikonabscheidung auf Haarfasern durch Kombination zweier spezifischer Silikontypen in anionische Tenside und kationische Polymere enthaltenden kosmetischen Reinigungsmitteln gesteigert werden kann. Dabei können die Silikone in üblichen Mengen eingesetzt werden - eine signifikante Erhöhung ihrer Einsatzmengen ist nicht erforderlich. Die spezifische Mischung zweier unterschiedlicher Silikontypen führt zu einer stabilen und nachhaltigen Ummantelung der Haarfasern, was zu einem langanhaltenden Pflegeeffekt, mehr Haarglätte sowie einem Schutz der Haarfasern vor mechanischen Schädigungen führt.

Behandelte Haarfasern weisen sehr gute Nass- und Trockenkämmbarkeiten, eine geringere Haarreibung bei der mechanischen Behandlung der Haare sowie mehr Weichheit und Geschmeidigkeit auf.

Die Reinigungsmittel sind zudem mild und weisen sowohl auf den Haarfasern als auch auf der Kopfhaut ein geringeres Austrocknungspotential auf als herkömmliche Pflegeshampoos.

Sie lassen sich außerdem sehr gut auf den Haarfasern verteilen und weisen hervorragende Schaumqualitäten auf.

Ein weiterer Vorteil ist, dass sich die zwei spezifischen Silikontypen als Premix - vorzugsweise als Emulsion - problemlos und ohne weitere Stabilisierungsmittel oder -schritte in die Reinigungsmittel einarbeiten lassen.

Ein erster Gegenstand der Erfindung ist ein kosmetisches Reinigungsmittel, enthaltend
a) mindestens ein anionisches Tensid
b) mindestens ein kationisches Polymer
c) mindestens ein Siloxanpolymer, welches mindestens drei unterschiedliche funktionelle Gruppen, ausgewählt aus Amino-, Aryl-, Alkoxy-, Hydroxyl-, Alkyl-, Acyl- und Hydroxyalkylgruppen enthält, und
d) mindestens ein Polydimethylsiloxan.

Bevorzugt ist das kosmetische Reinigungsmittel wässrig. Wässrige kosmetische Reinigungsmittel bezeichnen im Sinne der vorliegenden Erfindung eine Zusammensetzung, deren kosmetisch verträgliche Trägerkomponente Wasser, bevorzugt destilliertes Wasser, ganz besonders destilliertes Wasser ohne Wasserstoffperoxid (H₂O₂), ist. Der Anteil des Wassers in dem kosmetischen Reinigungsmittel beträgt bevorzugt 50 bis 95 Gew.-%, besonders bevorzugt 55 bis 94 Gew.-%, ganz besonders bevorzugt 60 bis 92,5 Gew.-%, noch mehr bevorzugt 65 bis 90 Gew.-%, bezogen auf den Gesamtanteil des kosmetischen Reinigungsmittels.

In einer besonders bevorzugten Ausführungsform der Erfindung umfasst das kosmetische Reinigungsmittel so viel Wasser, dass die übrigen in der Zusammensetzung enthaltenen Komponenten auf 100 Gew.-%, gemessen am Gesamtgewicht des Reinigungsmittels, komplettiert werden, auch bezeichnet als "ad 100".

Die Summe aller Komponenten des kosmetischen Reinigungsmittels beträgt 100 Gew.-%, bezogen auf den Gesamtanteil des kosmetischen Reinigungsmittels.

Bevorzugte Reinigungsmittel im Sinne der vorliegenden Erfindung sind Shampoos, Duschgele, Haarreinigungsspülungen, sowie Seifen und Badezusätze. Besonders bevorzugt handelt es sich bei den erfindungsgemäßen Reinigungsmitteln um Shampoos.

Als erste wesentliche Komponente enthalten die erfindungsgemäßen Reinigungsmittel mindestens ein anionisches Tensid a). Darunter sind prinzipiell alle für die Verwendung am menschlichen Körper geeigneten anionischen oberflächenaktiven Stoffe zu verstehen. Diese sind gekennzeichnet durch eine wasserlöslich machende, anionische Gruppe wie z.B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein.

Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkyl-gruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel 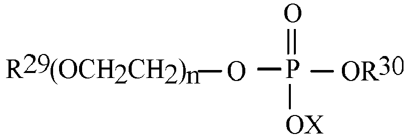 in der R29 bevorzugt für einen aliphatischen Kohlenwasserstoffrest mit 8 bis 30 Kohlenstoffatomen, R30 für Wasserstoff, einen Rest (CH₂CH₂O)ₙR29 oder X, n für Zahlen von 1 bis 10 und X für Wasserstoff, ein Alkali- oder Erdalkalimetall oder NR31 R32R33R34, mit R31 bis R34 unabhängig voneinander stehend für einen C1 bis C4-Kohlenwasserstoffrest, steht,
- sulfatierte Fettsäurealkylenglykolester der Formel

   R35CO(AlkO)ₙSO₃M

   in der R35CO- für einen linearen oder verzweigten, aliphatischen, gesättigten und/oder ungesättigten Acylrest mit 6 bis 22 C-Atomen, Alk für CH2CH2, CHCH3CH2 und/oder CH2CHCH3, n für Zahlen von 0,5 bis 5 und M für ein Kation steht,
- Monoglyceridsulfate und Monoglyceridethersulfate der Formel (III): 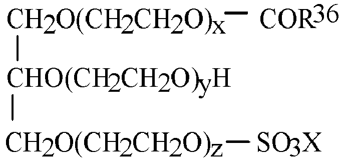 in der R36CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (III) eingesetzt, in der R36CO für einen linearen Acylrest mit 8 bis 18 Kohlenstoffatomen steht.

Bevorzugte anionische Tenside a) sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten, bevorzugt Natriumlaurylethersulfat mit durchschnittlich 2 Ethylenoxideinheiten (INCI-Bezeichnung: Sodium Laureth Sulfate).

Weiterhin besonders bevorzugte anionische Tenside sind geradkettige oder verzweigte Alkylsulfate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate.

In einer besonders bevorzugten Ausführungsform enthalten die erfindungsgemäßen kosmetischen Reinigungsmittel als anionisches Tensid a) mindestens 90 Gew.-% Alkyl(ether)sulfate, bezogen auf das Gesamtgewicht aller anionischen Tenside.

Das oder die anionischen Tenside a) werden in den erfindungsgemäßen Reinigungsmitteln vorzugsweise in einem Gewichtsanteil von 5-20 Gew.-% am Gesamtgewicht des Reinigungsmittels eingesetzt. Mehr bevorzugt sind Gewichtsanteile von 6-19 Gew.-%, besonders bevorzugt 7-17,5 Gew.-% ganz besonders bevorzugt 7,5-16 Gew.-% und insbesondere 7,5-15 Gew.-%.

In einer besonders bevorzugten Ausführungsform enthalten erfindungsgemäße Reinigungsmittel Alkyl(ether)sulfate in den zuvor genannten Mengen.

Als zweite wesentliche Komponente enthalten die erfindungsgemäßen Reinigungsmittel mindestens ein kationisches Polymer b).

Die Anwesenheit mindestens eines kationischen Polymeren b) in den erfindungsgemäßen Reinigungsmitteln unterstützt die stabilere und nachhaltigere Abscheidung höherer Mengen der spezifischen Silikone c) und d) auf den Haarfasern, wodurch ein gesteigerter Pflegeeffekt erzielt werden kann. Neben den bereits genannten Vorteilen unterstützt ein Gehalt an kationischen Polymeren b) in den erfindungsgemäßen Reinigungsmitteln zudem den guten Haargriff und ein erhöhtes Haarvolumen der behandelten Haarfasern.

Unter geeigneten kationischen Polymeren b) sind beispielsweise zu verstehen:
- quaternisierte Cellulosepolymere, beispielsweise Polyquaternium-10, wie sie unter den Bezeichnungen Celquat^{®} oder Polymer JR^{®} im Handel erhältlich sind
- hydrophob modifizierte Cellulosederivate, beispielsweise die unter dem Handelsnamen SoftCat^{®} vertriebenen kationischen Polymere
- kationische Alkylpolyglycoside
- kationiserter Honig, beispielsweise das Handelsprodukt Honeyquat^{®} 50
- kationische Guar-Derivate, wie beispielsweise die unter den Handelsnamen Cosmedia^{®} Guar, N-Hance^{®}, Jaguar^{®} und Polycare^{®} vertriebenen Produkte
- polymere Dimethyldiallylammoniumsalze und deren Copolymere mit Estern und Amiden von Acrylsäure und Methacrylsäure, vor allem Polyquaternium-6 und Polyquaternium-7. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich
- Vinylpyrrolidon-Vinylimidazoliummethochlorid-Copolymere, wie sie unter den Bezeichnungen Luviquat^{®} FC 370, FC 550, FC 905 und HM 552 angeboten werden
- quaternierter Polyvinylalkohol
- sowie die unter den Bezeichnungen Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Für die Verwendung in den erfindungsgemäßen Reinigungsmitteln sind kationische Guar Polymere besonders geeignet, beispielsweise unter den INCI-Bezeichnungen Guar Hydroxypropyltrimonium Chloride und/oder Hydroxypropyl Guar Hydroxypropyltrimonium Chloride bekannte Verbindungen. Es wurde gefunden, dass ganz besonders kationische Guar Polymere mit höheren Molgewichten und niedrigeren kationischen Ladungsdichten besonders geeignet sind.

Unter "höheren Molgewichten" sind in diesem Zusammenhang vorzugsweise mittlere Molekulargewichte der kationischen Guar-Polymere im Bereich von etwa 500.000 - 4.000.000 g/mol, mehr bevorzugt 750.000 - 3.500.000 g/mol und insbesondere 1.000.000 - 3.000.000 g/mol zu verstehen.

Unter "niedrigeren kationischen Ladungsdichten" sind in diesem Zusammenhang vorzugsweise kationische Ladungsdichten der kationischen Guar-Polymere im Bereich von etwa 0,1 - 1,5 meq/g, mehr bevorzugt 0,1 - 1 meq/g und insbesondere 0,1 - 0,75 meq/g zu verstehen.

Besonders bevorzugt sind unter den INCI-Bezeichnungen Guar Hydroxypropyltrimonium Chloride und/oder Hydroxypropyl Guar Hydroxypropyltrimonium Chloride bekannte kationische Guar-Polymere, welche mittlere Molgewichte und kationische Ladungsdichten in den zuvor genannten Bereichen aufweisen. Entsprechende Handelsprodukte sind bekannt und erhältlich, beispielsweise die unter den Handelsbezeichnungen Jaguar^{®} C14S und Jaguar^{®} C162.

Eine bevorzugte Ausführungsform der Erfindung ist dadurch gekennzeichnet, dass sie als kationisches Polymer b) mindestens ein kationisches Guar Polymer enthält.

Für eine besonders optimale Silikonabscheidung ist es im Sinne der vorliegenden Erfindung bevorzugt, wenn mindestens zwei unterschiedliche Guar Polymere eingesetzt werden; vorzugsweise physiologisch verträgliche und zuvor definierte Guar Hydroxypropyltrimonium- und Hydroxypropyl Guar Hydroxypropyltrimoniumsalze.

Eine weitere bevorzugte Ausführungsform der Erfindung ist dadurch gekennzeichnet, dass die Reinigungsmittel als kationisches Polymer b) physiologisch verträgliche Guar Hydroxypropyltrimonium- und Hydroxypropyl Guar Hydroxypropyltrimoniumsalze enthalten.

Der Gewichtsanteil des mindestens einen kationischen Polymeren b) am Gesamtgewicht des kosmetischen Reinigungsmittels beträgt vorzugsweise 0,01-5 Gew.-%, mehr bevorzugt 0,025-4 Gew.-%, besonders bevorzugt 0,04-3 Gew.-%, ganz besonders bevorzugt 0,05-2 Gew.-% und insbesondere 0,05 bis 1 Gew.-%.

In einer besonders bevorzugten Ausführungsform enthalten erfindungsgemäße Reinigungsmittel kationische Guar Polymere in den zuvor genannten Mengen, insbesondere physiologisch verträgliche Guar Hydroxypropyltrimonium- und Hydroxypropyl Guar Hydroxypropyltrimoniumsalze.

Als dritte wesentliche Komponente enthalten die erfindungsgemäßen Reinigungsmittel mindestens ein Siloxanpolymer c), welches mindestens drei unterschiedliche funktionelle Gruppen, ausgewählt aus Amino-, Aryl-, Alkoxy-, Hydroxyl-, Alkyl-, Acyl- und Hydroxyalkylgruppen, enthält.

Siloxane oder Siloxanpolymere sind Makromoleküle, die aus einem Polymerrückgrat aus alternierenden Silizium- und Sauerstoffatomen (Siloxanbindungen) bestehen, einschließlich organischer Seitengruppen wie beispielsweise die zuvor genannten. Die Anzahl der sich wiederholenden Siloxaneinheiten kann von einer bis zu mehreren Tausend reichen. Durch Anpassung der Länge der -Si-O-Kette, der Funktionalität der Seitengruppen und der Vernetzung zwischen Molekülketten können Silikone zu einer nahezu unendlichen Vielfalt von Materialien mit jeweils einzigartigen chemischen Eigenschaften und Leistungsmerkmalen synthetisiert werden. Für die Verwendung in den erfindungsgemäßen Reinigungsmitteln geeignete Siloxanpolymere c) sind netzwerkbildende Silikon Crosspolymere, die mindestens drei unterschiedliche funktionelle Gruppen, ausgewählt aus Aminogruppen, Arylgruppen, Alkoxygruppen, Hydroxylgruppen, Acylgruppen, Alkylgruppen und/oder Hydroxyalkylgruppen, enthalten.

Besonders bevorzugte funktionelle Gruppen sind primäre oder sekundäre Aminogruppen, gegebenenfalls substituierte Phenylgruppen, Methoxy-, Ethoxy-, Propoxy-, Phenoxygruppen, C₁-C₂₄-Acylgruppen, C₁-C₂₄-Alkylgruppen, insbesondere Methyl-, Ethyl-, Propyl-, Isopropyl-, Butyl-, Isobutylgruppen, C₂-C₈-Hydroxyalkylgruppen.

Es wurde gefunden, dass eine besonders zufriedenstellende Silikonabscheidung auf den Haarfasern erzielt werden kann, wenn als Siloxanpolymer c) netzwerkbildende Amodimethicone Crosspolymere verwendet werden, d.h. Aminogruppen und mindestens zwei weitere der zuvor genannten funktionellen Gruppen enthaltende Siloxanpolymere.

Besonders bevorzugt umfassen erfindungsgemäße Siloxanpolymere c) vier der zuvor genannten funktionellen Gruppen; besonders bevorzugt Amino-, Alkoxy-, Hydroxyl- und Alkylgruppen und insbesondere bevorzugt Amino-, Methoxy-, Hydroxyl- und Methylgruppen. Entsprechende netzwerkbildende Amodimethicone Crosspolymere sind unter der INCI-Bezeichnung Bis-Hydroxy/Methoxy/Methyl Amodimethicone Crosspolymer bekannt und erhältlich.

Der Gewichtsanteil des mindestens einen Siloxanpolymers c) am Gesamtgewicht des kosmetischen Reinigungsmittels beträgt vorzugsweise 0,01-5 Gew.-%, mehr bevorzugt 0,025-4 Gew.-%, besonders bevorzugt 0,04-3 Gew.-%, ganz besonders bevorzugt 0,05-2 Gew.-% und insbesondere 0,05 bis 1 Gew.-%.

In einer besonders bevorzugten Ausführungsform enthalten erfindungsgemäße Reinigungsmittel mindestens ein Siloxanpolymer c), welches Amino-, Alkoxy-, Hydroxyl- und Alkylgruppen, ganz besonders bevorzugt Amino-, Methoxy-, Hydroxyl- und Methylgruppen enthält.

Innerhalb dieser Ausführungsform sind insbesondere unter der INCI-Bezeichnung Bis-Hydroxy/Methoxy/Methyl Amodimethicone Crosspolymer bekannte Siloxanpolymere c) bevorzugt.

Als vierte wesentliche Komponente enthalten die erfindungsgemäßen Reinigungsmittel mindestens ein Polydimethylsiloxan d). Es wurde gefunden, dass die Kombination der Silikone c) und d) in den erfindungsgemäßen Reinigungsmitteln nicht nur eine Steigerung der Pflegeeffekte auf den Haarfasern gewährleistet, sondern zudem auch vorteilhaft für die Verarbeitbarkeit beider Silikone sowie deren stabile Inkorporation in die erfindungsgemäßen Reinigungsmittel ist. Die Polydimethylsiloxane d) werden in dem Netzwerk der Siloxanpolymere c) gehalten und aus den erfindungsgemäßen Reinigungsmitteln auf die Haarfasern aufgebracht. Dadurch wird eine bessere Haftung auf den Haarfasern erzielt.

Unter geeigneten Polydimethylsiloxanen d) (Dimethiconen) für die Verwendung in den erfindungsgemäßen Reinigungsmitteln sind Dimethicone einer Viskosität im Bereich von 1 - 100,000 cPs zu verstehen.

Der Gewichtsanteil des mindestens einen Polydimethylsiloxans d) am Gesamtgewicht des kosmetischen Reinigungsmittels beträgt vorzugsweise 0,01-5 Gew.-%, mehr bevorzugt 0,05-4 Gew.-%, besonders bevorzugt 0,1-3 Gew.-%, ganz besonders bevorzugt 0,15-2 Gew.-% und insbesondere 0,2 bis 1 Gew.-%.

In einer bevorzugten Ausführungsform werden als Polydimethylsiloxan d) Dimethicone einer Viskosität im Bereich von 1 - 100,000 cPs in den zuvor genannten Mengen eingesetzt.

Für einige Ausführungsformen kann es von besonderem Vorteil sein, wenn die Silikone c) und d) nicht einzeln in die erfindungsgemäßen kosmetischen Reinigungsmittel eingearbeitet werden, sondern als geeigneter Premix. Die Einarbeitung eines geeigneten Premix bietet den Vorteil, dass die Stabilisierung der Reinigungsmittel erleichtert wird und/oder energetisch aufwändige Schritte bei deren Herstellung reduziert bzw. vermieden werden können.

Vorteilhafte Ausführungsformen für geeignete, die Silikone c) und d) umfassende Premixe sind Emulsionen. Diese lassen sich problemlos durch Hinzufügen und Mischen in die Reinigungsmittel einarbeiten und erfordern weder erhöhte Temperaturen noch den Zusatz weiterer Stabilisierungsmittel.

Geeignete Emulsionen enthalten neben den Silikonen c) und d) vorzugsweise Wasser als Basis. Für die Stabilisierung der Emulsionen und/oder zur Verhinderung von Separationserscheinungen ist es vorteilhaft, wenn die Emulsionen zusätzlich Emulgatoren, ausgewählt aus anionischen, kationischen und/oder nichtionischen Emulgatoren, enthalten.

Bevorzugt enthalten geeignete Emulsionen nichtionische Emulgatoren wie beispielsweise alkoxylierte Fettalkohole und/oder Fettsäureester.

Unter alkoxylierten Fettalkoholen sind im Sinne der vorliegenden Erfindung lineare oder verzweigte, gesättigte oder ungesättigte C₈-C₂₄-Alkohole mit einem Alkoxylierungsgrad von 1-40 zu verstehen. Bevorzugt sind lineare C₁₀-C₁₈-Alkohole wie beispielsweise Decanol, Undecanol, Dodecanol (Laurylalkohol), Tridecanol, Tetradecanol (Myristylalkohol), Pentadecanol, Hexadecanol (Cetylalkohol), Heptadecanol, Octadecanol (Stearylalkohol) sowie Mischungen davon, die mit 1-40, bevorzugt 1-30, besonders bevorzugt 1-20 und insbesondere 2-20 Mol Ethylenoxid ethoxyliert wurden. Bevorzugte Beispiele für zuvor genannte Fettalkoholethoxylate sind beispielsweise Deceth-5, Deceth-7, Undeceth-7, Laureth-2, Laureth-4, Laureth-5, Laureth-7, Laureth-8, Laureth-9, Laureth-10, Laureth-12, Trideceth-5, Trideceth-7, Trideceth-9, Trideceth-10, Myreth-8, Myreth-10, Myreth-12, Ceteth-2, Ceteth-12, Ceteth-20, Seareth-2, Steareth-4, Steareth-12, Steareth-20, Ceteareth-12, Ceteareth-20 sowie Mischungen davon.

In einer besonders bevorzugten Ausführungsform werden die Silkikone c) und d) den erfindungsgemäßen Reinigungsmitteln in Form einer Emulsion E hinzugefügt, die weiterhin Wasser umfasst.

In einer weiteren besonders bevorzugten Ausführungsform werden die Silkikone c) und d) den erfindungsgemäßen Reinigungsmitteln in Form einer Emulsion hinzugefügt, die weiterhin Wasser sowie einen nichtionischen Emulgator umfasst.

Innerhalb dieser Ausführungsform ist es von Vorteil, wenn der nichtionische Emulgator aus linearen oder verzweigten, gesättigten oder ungesättigten C₈-C₂₄-Alkoholen mit einem Alkoxylierungsgrad von 1-40, vorzugsweise aus linearen C₁₀-C₁₈-Alkoholen, die mit 1-40 Mol Ethylenoxid ethoxyliert wurden, ausgewählt wird.

Eine für die Verwendung in den erfindungsgemäßen Mitteln besonders bevorzugte Emulsion E ist im Handel unter der INCI-Bezeichnung Dimethicone, Bis-Hydroxy/Methoxy/Methyl Amodimethicone Crosspolymer, Trideceth-10 bekannt und beispielsweise unter der Handelbezeichnung Belsil^{®} DADM 3240 E erhältlich.

Für den Fall, dass bei der Herstellung der erfindungsgemäßen Reinigungsmittel eine Emulsion E verwendet wird, so beträgt deren Einsatzmenge (bezogen auf das Gesamtgewicht des kosmetischen Reinigungsmittels) vorzugsweise 0,05-10 Gew.-%, mehr bevorzugt 0,1-9 Gew.-%, besonders bevorzugt 0,2-7,5 Gew.-% und insbesondere 0,5-5 Gew.-%.

Besonders bevorzugt wird eine unter der INCI-Bezeichnung Dimethicone, Bis-Hydroxy/Methoxy/Methyl Amodimethicone Crosspolymer, Trideceth-10 bekannte Emulsion in den zuvor genannten Mengen in den erfindungsgemäßen Reinigungsmitteln eingesetzt.

Neben den zuvor definierten wesentlichen Inhaltsstoffen können die erfindungsgemäßen Reinigungsmittel zusätzliche fakultative Wirk- und Inhaltsstoffe umfassen, die ihnen weitere vorteilhafte Eigenschaften verleihen.

Bevorzugte fakultative Inhaltsstoffe im Sinne der vorliegenden Erfindung sind einerseits amphotere und/oder zwitterionische Tenside zur Stabilisierung und/oder Optimierung der Schaumeigenschaften und/oder zur Steigerung des Reinigungspotentials und/oder der Milde der kosmetischen Reinigungsmittel sowie andererseits pflanzliche Öle zur weiteren Steigerung und/oder Unterstützung der Pflegeeigenschaften der kosmetischen Reinigungsmittel.

Unter geeigneten amphoteren oder zwitterionischen Tensiden sind Tenside zu verstehen, die sowohl eine kationische als auch eine anionische Ladung im Molekül tragen. Bevorzugt weisen amphotere Tenside mindestens eine quartäre Ammoniumgruppe und mindestens eine -COO⁻ - oder -SO₃⁻ - Gruppe, neben einer bevorzugt C8-C24-Alkyl- oder -Acylgruppe, im Molekül auf. Ferner sind sie befähigt, innere Salze auszubilden. Geeignete amphotere Tenside sind beispielsweise Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinat, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Bevorzugte amphotere und/oder zwitterionische Tenside sind die unter den INCI-Bezeichnungen Cocamidopropyl Betaine, Coco Betaine, Lauryl Betaine bekannten Betaine sowie die Salze des Cocoampho(di)acetats zu verstehen.

Besonders bevorzugt ist Cocamidopropyl Betaine.

Der Gewichtsanteil des oder der amphoteren und/oder zwitterionischen Tensids(e) am Gesamtgewicht des kosmetischen Reinigungsmittels beträgt vorzugsweise 0,5-10 Gew.-%, mehr bevorzugt 0,5-8 Gew.-%, besonders bevorzugt 0,5-7,5 Gew.-%, ganz besonders bevorzugt 0,5-6 Gew.-% und insbesondere 0,5 bis 5 Gew.-%.

Unter geeigneten pflanzlichen Ölen sind beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl zu verstehen.

Besonders bevorzugt sind Avocadoöl, Aprikosenkernöl, Hagebuttenkernöl, Jojobaöl, Kakaobutter, Mandelöl, Olivenöl, Pfirsichkernöl, Sheabutter, Sonnenblumenöl und Traubenkernöl.

Der Gewichtsanteil des oder der pflanzlichen Öls(e) am Gesamtgewicht des kosmetischen Reinigungsmittels beträgt vorzugsweise 0,01-5 Gew.-%, mehr bevorzugt 0,01-4 Gew.-%, besonders bevorzugt 0,025-3 Gew.-%, ganz besonders bevorzugt 0,025-2 Gew.-% und insbesondere 0,03 bis 1 Gew.-%.

Zur weiteren Steigerung der Milde und/oder der Schaumeigenschaften der erfindungsgemäßen kosmetischen Reinigungsmittel kann es in einer weiteren bevorzugten Ausführungsform von Vorteil sein, wenn die Reinigungsmittel - bezogen auf ihr Gewicht - zusätzlich 0,05 bis 10 Gew.-%, besonders bevorzugt 0,1 bis 7,5 Gew.-% und insbesondere 0,25 bis 5 Gew.-% mindestens eines nichtionischen Tensids enthalten.

Zu den erfindungsgemäß geeigneten nichtionischen Tensiden/Emulgatoren zählen bevorzugt
- die bereits zuvor offenbarten Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- Ethylenoxid- und Polyglycerin-Anlagerungsprodukte an Methylglucosid-Fettsäureester, Fettsäurealkanolamide und Fettsäureglucamide,
- Aminoxide,
- Sorbitanfettsäureester und Anlagerungeprodukte von Ethylenoxid an Sorbitanfettsäureester wie beispielsweise Polysorbate,
- Fettsäurealkanolamide der nachfolgenden allgemeinen Formel 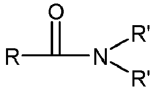 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Zuckerfettsäureester und Anlagerungsprodukte von Ethylenoxid an Zuckenfettsäureester,
- Anlagerungsprodukte von Ethylenoxid an Fettsäurealkanolamide und Fettamine,
- Alkyl(oligo)glucoside,
- Gemische aus Alkyl(oligo)glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden.

Geeignete Alkyl(oligo)glycoside können ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet.

Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann.

Besonders bevorzugte Alkyl(oligo)glycoside weisen einen Oligomerisierungsgrad zwischen 1,2 und 1,5 auf. Der Rest R steht bevorzugt für mindestens einen Alkyl- und/oder Alkenylrest mit 4 bis 24 C-Atomen. Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen oder Mischungen davon.

Neben den zuvor genannten wesentlichen und fakultativen Bestandteilen können die erfindungsgemäßen kosmetischen Reinigungsmittel weitere Inhaltsstoffe enthalten, die ihnen vorteilhafte Eigenschaften verleihen bzw. ihre vorteilhaften Eigenschaften unterstützen oder verstärken. Unter geeigneten weiteren Bestandteilen sind beispielsweise
∘ Emollientien (Weichmacher),
∘ Proteinhydrolysate,
∘ Vitamine,
∘ Antischuppenmittel,
∘ oder Mischungen davon zu verstehen.

Unter geeigneten Emollientien werden im Sinne der vorliegenden Erfindung vorzugsweise hydrophile Emollientien verstanden. Bevorzugte hydrophile Emollientien sind ethoxylierte Glycerinester, vorzugsweise C₈-C₃₀-Fettsäuremono- und -diester von Anlagerungsprodukten von 1 bis 30 Mol Ethylenoxid an Glycerin. Besonders bevorzugt ist ein der INCI-Bezeichnung PEG-7 Glyceryl Cocoate bekanntes hydrophiles Rückfettungsmittel.

PEG-7 Glyceryl Cocoate ist wasserlöslich und muss in einer wässrigen Basis nicht zusätzlich stabilisiert werden. Darüber hinaus verleiht es Haut und Haaren mehr Geschmeidigkeit und kann die Pflegewirkung der erfindungsgemäßen kosmetischen Reinigungsmittel unterstützen.

Für die Verwendung in den erfindungsgemäßen kosmetischen Reinigungsmitteln ist PEG-7 Glyceryl Cocoate zudem besonders geeignet, da es die Schaumqualität der Mittel verbessert und zur Emulgierung von Ölen oder Parfumölen beiträgt.

Der mindestens eine hydrophile Weichmacher kann in den erfindungsgemäßen kosmetischen Reinigungsmitteln vorzugsweise in einem Gewichtsanteil von 0,01 - 1,5 Gew.-% am Gesamtgewicht des kosmetischen Reinigungsmittels eingesetzt werden. Bevorzugt sind Einsatzmengen des mindestens einen hydrophilen Weichmachers von 0,02 - 1,4 Gew.-%, mehr bevorzugt 0,025 - 1,25 Gew.-% und insbesondere 0,03 - 1,1 Gew.-%.

In einer besonders bevorzugten Ausführungsform wird PEG-7 Glyceryl Cocoate in den zuvor genannten Mengen in den erfindungsgemäßen kosmetischen Reinigungsmitteln eingesetzt.

Unter geeigneten Proteinhydrolysaten sind Produktgemische zu verstehen, die durch sauer, basisch oder enzymatisch katalysierten Abbau von Proteinen (Eiweißen) erhalten werden können.

Es können Proteinhydrolysate pflanzlichen, tierischen und/oder marinen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Bevorzugte Beispiele für die kationischen Proteinhydrolysate und -derivate sind die unter den INCI - Bezeichnungen bekannten und im Handel erhältlichen Produkte: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Der Gewichtsanteil des oder der Proteinhydrolysats(e) am Gesamtgewicht der kosmetischen Reinigungsmittel beträgt bevorzugt 0,001 bis 1 Gew.-%, mehr bevorzugt 0,005 bis 1 Gew.-% und insbesondere 0,01 bis 1 Gew.-%.

Unter geeigneten Vitaminen sind bevorzugt die folgenden Vitamine, Provitamine und Vitaminvorstufen sowie deren Derivate zu verstehen:
- Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
- Vitamin B: zur Vitamin B-Gruppe oder zu dem Vitamin B-Komplex gehören u. a.
   ➢ Vitamin B₁ (Thiamin)
   ➢ Vitamin B₂ (Riboflavin)
   ➢ Vitamin B₃. Unter dieser Bezeichnung werden häufig die Verbindungen Nicotinsäure und Nicotinsäureamid (Niacinamid) geführt.
   ➢ Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols, Pantolacton sowie kationisch derivatisierte Panthenole. Einzelne Vertreter sind beispielsweise das Panthenoltriacetat, der Panthenolmonoethylether und dessen Monoacetat sowie kationische Panthenolderivate.
   ➢ Vitamin B₆ (Pyridoxin sowie Pyridoxamin und Pyridoxal).
- Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol).
- Vitamin F: unter dem Begriff "Vitamin F" werden üblicherweise essentielle Fettsäuren, insbesondere Linolsäure, Linolensäure und Arachidonsäure, verstanden.
- Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Besonders bevorzugt sind Vitamine, Provitamine und Vitaminvorstufen aus den Gruppen A, B, E und H. Insbesondere bevorzugt sind Nicotinsäureamid, Biotin, Pantolacton und/oder Panthenol.

Der Gewichtsanteil des oder der Vitamins(e), Vitaminderivats(e), und/oder der Vitaminvorstufe(n) am Gesamtgewicht des kosmetischen Reinigungsmittels beträgt bevorzugt 0,001 bis 1 Gew.-%, besonders bevorzugt 0,005 bis 1 Gew.-% und insbesondere 0,01 bis 0,5 Gew.-%.

Es wurde festgestellt, dass die erfindungsgemäßen Reinigungsmittel auch für eine Anwendung als Antischuppenzubereitung geeignet sind.

Der Gewichtsanteil an Antischuppenmitteln am Gesamtgewicht der Reinigungsmittel kann bevorzugt 0,01 bis 1 Gew.-%, mehr bevorzugt 0,025 bis 1 Gew.-%, besonders bevorzugt 0,05 bis 1 Gew.-% und insbesondere 0,075 bis 1 Gew.-% betragen.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten, Arnikaextrakten und/oder Propandiol Caprylate.

Bevorzugt sind Climbazol, Zink Pyrithion, Piroctone Olamine, Propandiol Caprylate oder Mischungen davon.

Weitere Wirk-, Hilfs- und Zusatzstoffe, die in den erfindungsgemäßen kosmetischen Reinigungsmitteln bevorzugt enthalten sein können, sind beispielsweise:
- Pflanzenextrakte,
- Perlglanzmittel,
- Trübungsmittel,
- Feuchthaltemittel,
- Parfums,
- UV-Filter,
- pH-Stellmittel wie NaOH, Zitronensäure, Milchsäure oder Mischungen davon,
- Strukturanten wie Maleinsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Wirkstoffe wie Bisabolol und/oder Allantoin,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konservierungsmittel, wie beispielsweise Natriumbenzoat oder Salicylsäure.

Ein zweiter Gegenstand der Erfindung ist die kosmetische Verwendung des Reinigungsmittels, wie zuvor definiert, für die Reinigung und Pflege von Haaren.

Besonders bevorzugt ist die kosmetische Verwendung des Reinigungsmittels, zuvor definiert, zur
- Verbesserung der Silikonabscheidung auf Haarfasern
- Erhöhung der Haarglätte
- Verminderung der Haarreibung bei mechanischen Behandlungen der Haarfasern
- Verbesserung der Kämmbarkeitseigenschaften.

Ein dritter Gegenstand der Erfindung ist ein kosmetisches Verfahren zur Reinigung und Pflege von Haaren, bei dem ein Reinigungsmittel, wie zuvor definiert, auf die - vorzugsweise nassen - Haare aufgebracht, einmassiert und nach einer Einwirkungszeit von 5 Sekunden bis 5 Minuten mit Wasser wieder ausgespült wird.

Für das erfindungsgemäße Verfahren und die erfindungsgemäße Verwendung gilt das zu den erfindungsgemäßen Mitteln Gesagte.

### Ausführungsbeispiele:

Die folgenden Beispiele sollen den Gegenstand der vorliegenden Erfindung erläutern, ohne ihn zu beschränken (Angaben in Gew.-% bezogen auf das Gesamtgewicht der kosmetischen Reinigungszusammensetzung, sofern nicht anders angegeben).

| | F1 | F2 | F3 | F4 | F5 | F6 |
|---|---|---|---|---|---|---|
| anionisches Tensid | 5-20 | 5-20 | | 5-20 | 5-20 | |
| Sodium Laureth Sulfate | | | 5-20 | | | 5-20 |
| kationisches Polymer | 0,01-5 | 0,01-5 | | 0,01-5 | 0,01-5 | |
| Guar Hydroxypropyltrimonium Chloride | | | 0,01-2 | | | 0,01-2 |
| Hydroxypropyl Guar Hydroxypropyltrimonium Chloride | | | 0,01-2 | | | 0,01-2 |
| Siloxanpolymer* | 0,01-5 | 0,01-5 | | | | |
| Bis-Hydroxy/Methoxy/Methyl Amodimethicone Crosspolymer | | | 0,01-5 | | | |
| Dimethylpolysiloxan | 0,01-5 | 0,01-5 | 0,01-5 | | | |
| Belsil^{®} DADM 3240 E** | | | | 0,05-10 | 0,05-10 | 0,05-10 |
| amphoteres Tensid | | 0,5-10 | | | 0,5-10 | |
| Cocamidopropylbetain | | | 0,5-5 | | | 0,5-5 |
| pflanzliches Öl | | 0,01-5 | | | 0,01-5 | |
| Aprikosenkernöl | | | 0,01-5 | | | 0,01-5 |
| Wasser und ggfs. weitere Hilfs- und Zusatzstoffe | ad 100 | ad 100 | ad 100 | ad 100 | ad 100 | ad 100 |

| | | | | | | |
|---|---|---|---|---|---|---|
| * Siloxanpolymer umfassend mindestens drei unterschiedliche funktionelle Gruppen, ausgewählt aus Amino-, Aryl-, Alkoxy-, Hydroxyl-, Alkyl-, Acyl- und Hydroxyalkylgruppen ** INCI-Bezeichnung: Aqua, Dimethicone, Bis-Hydroxy/Methoxy/Methyl Amodimethicone Crosspolymer, Trideceth-10 | | | | | | |

Es wurden die folgenden Zusammensetzungen F7 und F8 hergestellt. F7 ist ein Haarpflegeshampoo im Sinne der vorliegenden Erfindung; F8 ist eine Vergleichszusammensetzung und repräsentiert den Stand der Technik:

| | F7 | F8 |
|---|---|---|
| Sodium Laureth Sulfate | 12 | 12 |
| Guar Hydroxypropyltrimonium Chloride | 0,2 | 0,3 |
| Hydroxypropyl Guar Hydroxypropyltrimonium Chloride | 0,1 | |
| Belsil^{®} DADM 3240 E** | 1,2 | |
| Dimethicone 60.000 cSt | | 3 |
| Amodimethicone | | 0,25 |
| Cocamidopropylbetain | 1,5 | 1,5 |
| Aprikosenkernöl | 0,05 | 0,05 |
| Wasser und ggfs. weitere Hilfs- und Zusatzstoffe | ad 100 | ad 100 |

Gleiche Mengen der beiden Shampoos F7 und F8 wurden in einem Halbseitentest auf jeweils 10 Köpfen in üblicher und auf dieselbe Art aufgebracht und nach identischer Einwirkungszweit mit denselben Mengen an Wasser ausgespült. Anschließend wurden die Haare von 5 unabhängigen Experten hinsichtlich der Parameter
- Haargefühl
- Kämmbarkeit
- Glanz
- Volumen
- Bounce
- Gefühl der Haarspitzen
- Glätte
beurteilt, wobei die Benotungen (in ganzen Zahlen) von 1 (= schlecht) bis 5 (= sehr gut) vergeben werden konnten.

In allen zuvor genannten Parametern wurden Haare, welche mit der erfindungsgemäßen Zusammensetzung F7 gereinigt wurden, besser beurteilt als die mit der Vergleichszusammensetzung F8 gereinigten Haare.

Die Gesamtnoten waren
- 4,9 (für die mit der erfindungsgemäßen Zusammensetzung F7 gereinigten Haare)
- 4,7 (für die mit der Vergleichszusammensetzung F8 gereinigten Haare).

Darüber hinaus wiesen mit erfindungsgemäßen Reinigungsmitteln behandelte Haare eine geringere Haarreibung auf.

## Patentansprüche

1. Kosmetisches Reinigungsmittel, enthaltend
a) mindestens ein anionisches Tensid
b) mindestens ein kationisches Polymer
c) mindestens ein Siloxanpolymer, welches mindestens drei unterschiedliche funktionelle Gruppen, ausgewählt aus Amino-, Aryl-, Alkoxy-, Hydroxyl-, Alkyl-, Acyl- und Hydroxyalkylgruppen enthält, und
d) mindestens ein Polydimethylsiloxan.

2. Kosmetisches Reinigungsmittel nach Anspruch 1, enthaltend (bezogen auf sein Gesamtgewicht)
- 5-20 Gew.-% a)
- 0,01-5 Gew.-% b)
- 0,01-5 Gew.-% c)
- 0,01-5 Gew.-% d).

3. Kosmetisches Reinigungsmittel nach einem der vorhergehenden Ansprüche, enthaltend als anionisches Tensid a) mindestens ein Alkyl(ether)sulfat.

4. Kosmetisches Reinigungsmittel nach einem der vorhergehenden Ansprüche, enthaltend als kationisches Polymer b) mindestens ein kationisches Guar Polymer, vorzugsweise physiologisch verträgliche Guar Hydroxypropyltrimonium- und Hydroxypropyl Guar Hydroxypropyltrimoniumsalze.

5. Kosmetisches Reinigungsmittel nach einem der vorhergehenden Ansprüche, enthaltend ein Siloxanpolymer c), welches Amino-, Alkoxy-, Hydroxyl- und Alkylgruppen enthält, vorzugsweise Amino-, Methoxy-, Hydroxyl- und Methylgruppen und insbesondere ein unter der INCI-Bezeichnung Bis-Hydroxy/Methoxy/Methyl Amodimethicone Crosspolymer bekanntes Siloxanpolymer c).

6. Kosmetisches Reinigungsmittel nach einem der vorhergehenden Ansprüche, enthaltend als Polydimethylsiloxan d) Dimethicone einer Viskosität im Bereich von 1 - 100,000 cPs.

7. Kosmetisches Reinigungsmittel nach einem der vorhergehenden Ansprüche, enthaltend die Silikone c) und d) in einer Emulsion E, die weiterhin Wasser umfasst.

8. Kosmetisches Reinigungsmittel nach Anspruch 7, enthaltend die Silikone c) und d) in einer Emulsion E, die weiterhin Wasser und einen nichtionischen Emulgator umfasst.

9. Kosmetisches Reinigungsmittel nach einem der Ansprüche 7 oder 8, enthaltend eine Dimethicone, Bis-Hydroxy/Methoxy/Methyl Amodimethicone Crosspolymer, Trideceth-10 und Wasser umfassende Emulsion E.

10. Kosmetisches Reinigungsmittel nach einem der Ansprüche 7 bis 9, enthaltend die Emulsion E in einem Gewichtsanteil von 0,05-10 Gew.-% am Gesamtgewicht des Reinigungsmittels.

11. Kosmetisches Reinigungsmittel nach einem der vorhergehenden Ansprüche, enthaltend
- mindestens ein amphoteres Tensid in einem Gewichtsanteil von 0,5 - 10 Gew.-% am Gesamtgewicht des Reinigungsmittels und/oder
- mindestens ein pflanzliches Öl in einem Gewichtsanteil von 0,01 - 5 Gew.-% des Reinigungsmittels.

12. Kosmetische Verwendung eines Reinigungsmittels nach einem der Ansprüche 1 bis 11 zur Reinigung und Pflege von Haaren.

13. Kosmetische Verwendung nach Anspruch 12 zur
- Verbesserung der Silikonabscheidung auf Haarfasern
- Erhöhung der Haarglätte
- Verminderung der Haarreibung bei mechanischen Behandlungen der Haarfasern
- Verbesserung der Kämmbarkeitseigenschaften.

14. Kosmetisches Verfahren zur Reinigung und Pflege von Haaren, bei dem ein Reinigungsmittel nach einem der Ansprüche 1 bis 11 auf die - vorzugsweise nassen - Haare aufgebracht, einmassiert und nach einer Einwirkungszeit von 5 Sekunden bis 5 Minuten mit Wasser wieder ausgespült wird.
